# EUROPEAN PATENT SPECIFICATION

(11) **EP 0 592 627 B1**
(45) Date of publication and mention of the grant of the patent: **04.06.1997**
(21) Application number: 93907618.8
(22) Date of filing: 19.03.1993
(51) Int. Cl.: C07C 37/62, C07C 37/70, C07C 37/82, C07C 39/367

(54) **PROCESS FOR HIGH PURITY TETRABROMOBISPHENOL-A**
VERFAHREN ZUR HERSTELLUNG VON HOCHREINEM TETRABROMBISPHENOL-A
PROCEDE POUR OBTENIR DU TETRABROMOBISPHENOL-A DE PURETE ELEVEE

(30) Priority: 01.04.1992 US 861544; 15.12.1992 US 990414
(43) Date of publication of application: 20.04.1994
(73) Proprietor: ALBEMARLE CORPORATION, Richmond, Virginia 23219 (US)
(72) Inventor: McKINNIE, Bonnie, G., Magnolia, AR 71753 (US); SHARP, Gary, L., Magnolia, AR 71753 (US); WILLIAMS, Robert, Eric, Magnolia, AR 71753 (US)
(74) Representative: Sandmair, Kurt, Dr. Dr.
(86) International application number: PCT/US93/02585
(87) International publication number: WO 93/20031

(56) References cited:
- EP-A- 0 380 363
- EP-A- 0 472 395
- DE-A- 3 417 027
- DE-B- 1 268 149
- US-A- 3 029 291
- US-A- 4 628 124
- US-A- 4 701 568
- US-A- 5 107 035

## Description

This invention relates to processes for enhancing the purity of a tetrabromobisphenol-A flame retardant product.

### Background

4,4'-Isopropylidenebis(2,6-dibromophenol), otherwise known as tetrabromobisphenol-A (TBBPA), is a well-known commercial flame retardant which, as used, is typically an impure product. However, when intended for use as a flame retardant, it is preferably prepared so as to have a particularly low organic impurity content, e.g., by a process such as those disclosed in U.S. Patents 4,628,124, 4,783,556, 4.909,997, 5,017,728, 5,059,722, and 5,138,103. In at least most of these processes, TBBPA is recovered from the reaction mass by adding water to precipitate the product.

Although the TBBPA products obtained from the known processes are suitable for use in most flame retardant applications, the requirements for flame-retardant polymers and plastics to be used in the electronics industry provide a need for a TBBPA product having both a low organic impurity content and a low ionic impurity content.

### Summary of Invention

It has now been found that (1) the purity of a tetrabromobisphenol-A product can be enhanced by contacting it with a quality-enhancing amount of water having a resistivity >50,000 ohms and subsequently heat-treating it to provide a TBBPA product containing <20 ppm of total ionic impurity and (2) the treated product has increased value as a flame retardant.

The tetrabromobisphenol-A product subjected to this treatment may be a TBBPA prepared by any known process but is preferably a TBPPA having a low organic impurity content and most preferably a TBPPA prepared by (1) adding 3.9-4.2 molar proportions of bromine and optionally also a methanol solvent to a solution of one molar proportion of bisphenol-A in a methanol solvent while maintaining a reaction temperature in the range of 0-40°C, (2) optionally adding more methanol solvent, and (3) precipitating the TBPPA thus formed after bromination is substantially complete; the methanol solvent of steps (1) and (2) containing less than 5% by weight of water and being used in a total amount in the range of 25-43 molar proportions.

### Detailed Description

### 1. TBPPA Starting Material

As already indicated, the TBPPA product which is purified in the practice of the invention may be any TBPPA requiring such purification, i.e., a product which is predominantly TBPPA but also contains ionic impurities - the total ionic impurity in such a product being any one or more compounds represented by the formula MBr wherein M is hydrogen or an alkali, alkaline earth, or other metal ion such as a Ba, Ca, Fe, K, Na, Mg, or Mn ion, and usually being >50% by weight HBr. However, it is preferred that the product be one which has a low organic impurity content.

Among the preferred starting materials for the purification process of the invention are the products of the processes of U.S. Patents 4,783,556, 4,909,997, 5,017,728, and 5,059,722, all of which provide products which generally contain <3% by weight of the organic impurities apt to be found in TBPPA products, e.g., partially brominated bisphenol compounds such as mono-, di-, and tribromobisphenol-A; partially brominated phenols such as mono-, di-, and tribromophenol; brominated phenylphenols; and the 1-bromo-, 1,1-dibromo- and 1,3-dibromo-2-(3,5-dibromo-4-hydroxyphenyl)-2-methoxypropanes.

The most preferred TBPPA starting materials, however, are those prepared by (1) adding 3.94.2 molar proportions of bromine and optionally also a methanol solvent to a solution of one molar proportion of bisphenol-A in a methanol solvent while maintaining a reaction temperature in the range of 0-40°C, (2) optionally adding more methanol solvent, and (3) precipitating the TBPPA thus formed after bromination is substantially complete; the methanol solvent of steps (1) and (2) containing less than 5% by weight of water and being used in a total amount in the range of 25-43 molar proportions. Using this process to prepare TBPPA not only has the advantage of providing a product which is low in organic impurities but also substantially reduces the amount of methyl bromide by-product formed.

In this novel process for preparing particularly preferred TBPPA starting materials for the purification process, it is critical to observe the above-specified features regarding amount of solvent, water content, bromine/bisphenol-A ratio, and temperature in order to achieve the end of providing a large-scale process for producing a high yield of TBPPA having greatly reduced organic impurities. Thus:
(A) The methanol solvent, at least a portion of which is used to dissolve the bisphenol-A, must have a water content of < 5%. However, it preferably contains at least 1%, preferably 1.5-4%, and most preferably about 4% by weight of water. The presence of some water in the solvent is believed to decrease the amount of methyl bromide formed in the process by reacting with HBr to form H₃O⁺Br⁻.
(B) Whether the total amount of methanol solvent employed is used initially to dissolve the bisphenol-A or a portion of that solvent is reserved to be added together with or after the bromine, that total amount must be in the range of 2543 mols, preferably 25-35 mols, per mol of the bisphenol-A to be brominated, since the use of a smaller amount would lead to a decrease in the yield of the desired, completely brominated bisphenol-A, i.e., an increase in the amount of partially brominated byproducts. The desired effect of using the prescribed amount of methanol can be achieved when all of the methanol is used to dissolve the bisphenol-A initially. However, it is usually preferred to (1) employ only 75-90% of the methanol initially and then (2) add the remaining 25-10% of the methanol during the bromine feed step (as in U.S. Patents 5,017,728, 5,059,722, and 5,138,103) and/or after the bromine feed has been completed.
(C) The 3.9-4.2 molar proportions of bromine permit the use of (1) a slight stoichiometric deficit of bromine to reduce contaminination of the product with ionic impurities, (2) an exactly stoichiometric amount of bromine, or (3) a slight stoichiometric excess of bromine to increase the amount of completely brominated bisphenol-A obtained. Whether the bromine is fed as a liquid or as a vapor, it is preferably fed in diluted form - liquid bromine being dilutable with a portion of the methanol solvent, and vaporized bromine being dilutable with a gas such as nitrogen, steam, or argon. It has unexpectedly been found that the use of vaporized bromine, whether diluted or not, provides a TBPPA product having less impurities than the product obtained when liquid bromine is utilized.
(D) A reaction temperature in the range of 0-40°C, preferably 15-30°C, is employed to minimize the co-formation of methyl bromide (a particularly undesirable by-product from the environmental aspect). The use of such temperatures instead of higher temperatures, e.g., temperatures >50°C, reduces the rate at which the tetrabrominated product is formed, but it has been found possible to compensate for the the resultant failure of the process to effect complete bromination during the bromine feed by employing a cook time subsequent to the bromine addition step. The cook time varies with the particular amounts of solvent and bromine used, as well as with the bromination temperature, but can be readily determined empirically for any set of conditions. As an example, cook times of 0.5-2 hours have been found suitable when using 1-1.5% excess bromine, a methanol/bisphenol mol ratio of 33/1, and a temperature of 15-30°C.

The purity of the product can also be affected by the bromine feed rate. Thus, when liquid bromine is employed, this rate is preferably > 0.2, more preferably > 0.4, even more preferably at least 2.0, and most preferably at least 3-7 cm/second; and, when vaporized bromine is used, the linear velocity rate at which it is fed is preferably >85, preferably >500, and most preferably at least about 1800 cm/second. There is no real upper limit on the feed velocity for the bromine other than what is imposed by physical constraints of the process equipment or the ability to control reaction rate or temperature adequately.

While the degree of agitation of the reaction mass may also affect the purity of the product, this factor is less pronounced in industrial-scale reactions than in lab-scale ones.

After substantial completion of the bromination reaction, the product is separated from the reaction mass by conventional technqiues, such as adding sulfur dioxide to destroy any unreacted bromine and adding water to precipitate the TBPPA product, which contains < 2% by weight of organic impurities, although the total ionic impurity content is typically ≥60 ppm.

### 2. Reduction of Ionic Impurities

A key feature of this invention is the step of contacting a TBPPA product with a quality-enhancing amount of water having a resistivity >50,000 ohms prior to heat treating it. Such treated water can be obtained by known methods, i.e., contacting water with an ion-exchange resin, such as those which are commercially available, until the resistivity is > 50,000 ohms - the water having previously been treated in any one or more commercially-available reverse osmosis units when particularly high resistivities (e.g., resistivities >500,000 ohms or even >1,000,000 ohms) are desired.

The amount of treated water with which the TBPPA product is contacted may be any amount sufficient to enhance the quality of the product. However, it is preferably at least 0.1, more preferably at least 0.2, and most preferably 0.3-0.5 gram per gram of product. More treated water could be used, if desired; but economic considerations and manufacturing equipment limitations ordinarily militate against using more than required to enhance the quality of the product to the desired degree.

While the TBPPA product could beneficially be contacted with the treated water at any stage in its production, the contact is generally effected after the TBPPA has been synthesized, e.g., (A) during the procedure in which the TBPPA product is precipitated from the reaction mixture by the addition of water and then filtered or centrifuged from the slurry thus formed - preferably after by-product methyl bromide has been removed by any suitable technique - or (B) after the product has been dried. These alternatives include, *inter alia,* drying the product and grinding it to a powder having an average particle size of ≤70µ before contacting it with the treated water.

Another key feature of the invention is the step of heat treating the TBPPA product to reduce the amount of total ionic impurity in the product to <20 ppm, preferably <10 ppm, and even more preferably <5 ppm.

The temperature used for the heat treatment is generally >110°C, preferably 120-180°C, and most preferably 130-175°C. Higher or lower temperatures are also operable, although the temperature should not be high enough to cause melting or degradation of the TBPPA product; and temperatures lower than the preferred temperatures necessitate longer heating times.

The time required for the heat treatment varies with the temperature and is preferably > 10 seconds, more preferably 30 seconds to one hour, and most preferably 5-30 minutes at the preferred temperatures, although this time may also vary with the particular equipment used for the treatment. Any suitable equipment may be employed, including, e.g., the Wyssmont Turbo-Dryer® and a Bepex Torusdisc® Dryer such as Torusdisc® Model TDJ2611 having 218 square feet (20.3 m²) of heat transfer area and a 26 minute residence time at 120°C.

The following examples are given to illustrate the invention and are not intended as a limitation thereof. In these examples, a "Mitchell et al." refers to U.S. Patent 4,783,556 - a procedure of Mitchell et al. being the procedure of that patent, and a Mitchell et al. product being a TBPPA product of that procedure - and "plant process water" is untreated water containing 150-200 ppm total dissolved solids.

### COMPARATIVE EXAMPLE A

Prepare five TBPPA products by the general procedure of Mitchell et aL, feeding the bromine over a period of 65 minutes at a rate of 95.2 kg/minute and additional bromine at a rate of 47 kg/minute, and - after completion of the bromination - cooking the product at reflux for 30 minutes at a pressure of 0.1 MPa. Table 1 shows, for each run, the water content of the methanol used, the maximum temperature during the reaction and cook period, and the yield of methyl bromide formed, based on a theoretical yield of 0.7g of methyl bromide/g of tetrabromobisphenol-A formed.

**Table 1**

| Run # | H₂O Content (wt.%) | Maximum Temp. (°C) | Methyl Bromide (% yield) |
|---|---|---|---|
| 1 | 2 | 67.5 | 42.6 |
| 2 | 5 | 70.2 | 33.6 |
| 3 | 8 | 72.0 | 28.3 |
| 4 | 11 | 73.4 | 24.1 |
| 5 | 14 | 74.2 | 21.1 |

### EXAMPLE 1

Prepare two TBPPA products by a procedure using excess bisphenol-A to determine the rate at which methyl bromide is formed - specifically by adding 167g (1.04 mols) of bromine through a dip tube to a solution of 67g (0.29 mol) of bisphenol-A in 350 mL (8.64 mols) of methanol and 11.5 mL of water (a methanol having a water content of 4% by weight), stirring the reaction mixture at reaction temperature until the bromine feed has been completed, and cooking the mixture at the reaction temperature for an additional time during which samples are removed and analyzed by gas chromatography to determine the amount of methyl bromide formed. The bromine feed times and temperatures used, the minutes after bromine addition at which the samples were taken, and the results of the analysis are shown in Table 2.

**Table 2**

| Reaction Conditions | Run #1 | Run #2 |
|---|---|---|
| Temperature (°C) | 15 | 30 |
| Br₂ feed time (min.) | 15 | 12 |
| CH₃Br formed (% yield/ minutes after Br₂ added | 0.027%/8 min. 0.15%/35 min. 0.41%/95 min. 0.56%/140 min. | 0.42%/10 min. 1.38%/30 min. 1.90%/60 min. 2.70%/90 min. |

### EXAMPLE 2

Charge a suitable reaction vessel with 180g (0.79 mol) of bisphenol-A and 880 mL (21.7 mols) of methanol containing 4% by weight of water. Generate bromine vapor from 513g (3.2 mols) of bromine and feed the vapor to the reaction vessel over a period of 52 minutes while maintaining the temperature at 28-32°C by the use of an ice bath. After completing addition of the bromine, add another 175 mL (4.3 mols) of methanol containing 4% by weight of water to provide an overall methanol/bisphenol-A mol ratio of 32/1, and stir the mixture at 30°C. Sample the homogeneous mixture one hour after completing the bromine feed (at which time analysis shows the presence of 97.18% TBPPA, 2.79% tribromobisphenol-A, 0.03% dibromophenol, and 0.03% hydrolyzable impurities) and again 30 minutes later (at which time analysis shows the tribromobisphenol-A content to have been reduced to 2.17%). Then add 700 mL of water over 20 minutes and filter the resulting orange mixture.

### EXAMPLE 4

Wash the product of Example 3 with 500 mL of deionized water, place the isolated product in a crystallizing dish, and add 1.0 mL of 56% HBr. Then oven dry the product at 130 °C for four hours. The product thus obtained has a tetrabromobisphenol-A content of 99.24%, an ionic bromide content of 0 ppm, a hydrolyzable bromide content of 21 ppm, an acetone color of 10 APHA, and a TOHTO color of 20 APHA.

### EXAMPLE 5

Slurry 100g of a Mitchell et al. TBPPA product having 66 ppm total ionics in a solution of 20% by weight of methanol in deionized water having a resistivity of ∼1000,000 ohms. Then filter the TBPPA product from the slurry, wash it with 200 mL of deionized water having a resistivity of - 100,000 ohms, and heat-treat it 1t 140°C for 2.5 days. Analysis shows the total ionics to have been reduced to <2 ppm by the treatment.

### COMPARATIVE EXAMPLE B

Repeat Example 5 except for replacing the deionized water with plant process water both for the methanol solution and for the wash water. Analysis shows the total ionics to have been reduced only to 30 ppm by the treatment.

### EXAMPLE 6

Essentially repeat the procedure of Example 5 except for starting with a Mitchell et al. TBPPA product having 62 ppm total ionics, using a solution of 10% by weight of methanol in the deionized water, and heat-treating the washed product at 120°C for 22 hours. Analysis of the washed product prior to the heat treatment, after four hours heating, and after 22 hours heating show the total ionics at those stages to be 46 ppm, <8 ppm, and <2 ppm, respectively.

### COMPARATIVE EXAMPLE C

Repeat Example 6 except for conducting the heat treatment at 70°C. After 22 hours the total ionics content has been reduced only to 40 ppm.

## Claims

1. A process for enhancing the purity of a tetrabromobisphenol-A product, characterized in that the product is contacted with a quality-enhancing amount of treated water having a resistivity >50,000 ohms and subsequently heat-treated at a temperature >110°C to provide a tetrabromobisphenol-A product containing <20 ppm of total ionic impurity.

2. The process of claim 1 wherein the quality-enhancing amount of treated water is at least 0.2g/g of the tetrabromobisphenol-A product.

3. The process of claim 2 wherein the quality-enhancing amount of treated water is 0.3-0.5g/g of the tetrabromobisphenol-A product.

4. The process of claim 1 wherein the treated water has a resistivity >50,000 ohms.

5. The process of claim 4 wherein the treated water has a resistivity > 1,000,000 ohms.

6. The process of claim 1 wherein the heat treatment is conducted at a temperature in the range of 120-180°C.

7. The process of claim 1 wherein the tetrabromobisphenol-A product is a dry powder when it is contacted with the treated water.

8. The process of claim 1 wherein the heat treatment is continued until the total ionic impurity in the product is <10 ppm.

9. The process of claim 8 wherein the heat treatment is continued until the total ionic impurity in the product is <5 ppm.

10. The process of claim 1 wherein the tetrabromobisphenol-A product which is contacted with the quality-enhancing amount of treated water and then heat-treated is a tetrabromobisphenol-A product prepared by (1) adding 3.9-4.2 molar proportions of bromine and optionally also a methanol solvent to a solution of one molar proportion of bisphenol-A in a methanol solvent while maintaining a reaction temperature in the range of 0-40°C, (2) optionally adding more methanol solvent, and (3) precipitating the tetrabromobisphenol-A thus formed after bromination is substantially complete; the methanol solvent of steps (1) and (2) containing less than 5% by weight of water and being used in a total amount in the range of 25-43 molar proportions.

11. The process of claim 10 wherein the bromine is added as a vapor, the methanol contains about 4% by weight of water and is used in a total amount in the range of 25-35 mols/mol of bisphenol-A, and the reaction temperature is about 30°C.

## Patentansprüche

1. Verfahren zur Verbesserung der Reinheit eines Tetrabrombisphenol-A-Produkts, dadurch gekennzeichnet, daß das Produkt mit einer qualitätsverbessernden Menge von behandeltem Wasser mit einem Widerstand von mehr als 50.000 Ohm in Kontakt gebracht und anschließend bei einer Temperatur von mehr als 110°C wärmebehandelt wird, um ein Tetrabrombisphenol-A-Produkt mit insgesamt weniger als 20 ppm Ionenverunreinigung herzustellen.

2. Verfahren nach Anspruch 1, bei dem die qualitätsverbessernde Menge an behandeltem Wasser mindestens 0,2 g/g des Tetrabrombisphenol-A-Produkts beträgt.

3. Verfahren nach Anspruch 2, bei dem die qualitätsverbessernde Menge an behandeltem Wasser 0,3 bis 0,5 g/g des Tetrabrombisphenol-A-Produkts beträgt.

4. Verfahren nach Anspruch 1, bei dem das behandelte Wasser einen Widerstand von mehr als 50.000 Ohm hat.

5. Verfahren nach Anspruch 4, bei dem das behandelte Wasser einen Widerstand von mehr als 1.000.000 Ohm hat.

6. Verfahren nach Anspruch 1, bei dem die Wärmebehandlung bei einer Temperatur im Bereich von 120 bis 180°C durchgeführt wird.

7. Verfahren nach Anspruch 1, bei dem das Tetrabrombisphenol-A-Produkt ein trockenes Pulver ist, wenn es in Kontakt mit dem behandelten Wasser gebracht wird.

8. Verfahren nach Anspruch 1, bei dem die Wärmebehandlung fortgesetzt wird, bis die gesamte Ionenverunreinigung im Produkt weniger als 10 ppm beträgt.

9. Verfahren nach Anspruch 8, bei dem die Wärmebehandlung fortgesetzt wird, bis die gesamte Ionenverunreinigung im Produkt weniger als 5 ppm beträgt.

10. Verfahren nach Anspruch 1, bei dem das Tetrabrombisphenol-A-Produkt, das in Kontakt mit der qualitätsverbessernden Menge behandeltem Wasser gebracht und dann wärmebehandelt wird, wie folgt hergestellt wird:
(1) Zugabe von 3,9 bis 4,2 Molanteilen Brom und bei Bedarf außerdem einem Methanollösungsmittel zu einer Lösung aus einem Molanteil Bisphenol-A in einem Methanollösungsmittel, während die Reaktionstemperatur im Bereich von 0 bis 40°C gehalten wird;
(2) bei Bedarf Zugabe von mehr Methanollösungsmittel und
(3) Ausfällen des auf diese Weise hergestellten Tetrabrombisphenols-A, nachdem die Bromierung im wesentlichen abgeschlossen ist;
wobei das Methanollösungsmittel aus den Schritten (1) und (2) weniger als 5 Gew.-% Wasser enthält und in einer Gesamtmenge im Bereich von 25 bis 43 Molanteilen verwendet wird.

11. Verfahren nach Anspruch 10, bei dem das Brom als Dampf zugesetzt wird, das Methanol etwa 4 Gew.-% Wasser enthält und in einer Gesamtmenge von 25 bis 35 Mol/Mol Bisphenol A verwendet wird und die Reaktionstemperatur etwa 30°C beträgt.

## Revendications

1. Procédé pour améliorer la pureté d'un tétrabromobisphénol-A, caractérisé en ce que le produit est mis en contact avec une quantité améliorant la qualité d'une eau traitée ayant une résistivité supérieure à 50.000 ohms, puis traité par la chaleur à une température supérieure à 110 °C pour donner un tétrabromobisphénol-A contenant moins de 20 ppm d'impuretés ioniques totales.

2. Procédé selon la revendication 1, dans lequel la quantité améliorant la qualité de l'eau traitée est d'au moins 0,2 gramme/gramme du tétrabromobisphénol-A.

3. Procédé selon la revendication 2, dans lequel la quantité améliorant la qualité de l'eau traitée est de 0,3 à 0,5 gramme/gramme du tétrabromobisphénol-A.

4. Procédé selon la revendication 1, dans lequel l'eau traitée a une résistivité supérieure à 50.000 ohms.

5. Procédé selon la revendication 4, dans lequel l'eau traitée a une résistivité supérieure à 1.000.000 ohms.

6. Procédé selon la revendication 1, dans lequel le traitement thermique est effectué à une température dans l'intervalle de 120 à 180 °C.

7. Procédé selon la revendication 1, dans lequel le tétrabromobisphénol-A est une poudre sèche lorsqu'il est mis en contact avec l'eau traitée.

8. Procédé selon la revendication 1, dans lequel le traitement thermique est poursuivi jusqu'à ce que l'impureté ionique totale présente dans le produit soit inférieure à 10 ppm.

9. Procédé selon la revendication 8, dans lequel le traitement thermique est poursuivi jusqu'à ce que l'impureté ionique totale présente dans le produit soit inférieure à 5 ppm.

10. Procédé selon la revendication 1, dans lequel le tétrabromobisphénol-A qui est mis en contact avec la quantité améliorant la qualité de l'eau traitée, puis traité thermiquement est un tétrabromobisphénol-A préparé (1) en ajoutant des proportions de brome de 3,9 à 4,2 moles et facultativement aussi du méthanol comme solvant à une solution d'une proportion de 1 mole de bisphénol A dans du méthanol comme solvant tout en maintenant une température de réaction dans l'intervalle de 0 à 40 °C, (2) en ajoutant facultativement un supplément de méthanol comme solvant, et (3) en précipitant le tétrabromobisphénol-A ainsi formé après que la bromation est pratiquement complète ; le méthanol utilisé comme solvant dans les étapes (1) et (2) contenant moins de 5 % en poids d'eau et étant utilisé dans une quantité totale dans l'intervalle de 25 à 43 proportions molaires.

11. Procédé selon la revendication 10, dans lequel le brome est ajouté à l'état de vapeur, le méthanol contient environ 4 % en poids d'eau et est utilisé dans une quantité totale dans l'intervalle de 25 à 35 moles/mole de bisphénol A, et la température de réaction est d'environ 30 °C.
